## Europäisches Patentamt

## European Patent Office

## Office européen des brevets

(19)

(11) Veröffentlichungsnummer: **0 111 895**
**B1**

(12) **EUROPÄISCHE PATENTSCHRIFT**

(45) Veröffentlichungstag der Patentschrift:
15.10.86

(51) Int. Cl.⁴: **A 61 K 7/48**

(21) Anmeldenummer: **83112606.5**

(22) Anmeldetag: **15.12.83**

(54) **Hautpflegeemulsion.**

(30) Priorität: **23.12.82 DE 3247655**

(43) Veröffentlichungstag der Anmeldung:
**27.06.84 Patentblatt 84/26**

(45) Bekanntmachung des Hinweises auf die Patenterteilung:
**15.10.86 Patentblatt 86/42**

(84) Benannte Vertragsstaaten:
**AT BE CH DE FR GB IT LI LU NL SE**

(56) Entgegenhaltungen:
**EP-A-0 060 372**
**CH-A-270 135**
**DE-A-2 434 063**
**US-A-4 393 044**

(73) Patentinhaber: **Henkel Kommanditgesellschaft auf Aktien, Postfach 1100 Henkelstrasse 67, D-4000 Düsseldorf- Holthausen (DE)**

(72) Erfinder: **Ansmann, Achim, Dr., Fichtestrasse 19, D-4010 Hilden (DE)**
Erfinder: **Fischer, Karin, Ludwigstrasse 4, D-4000 Düsseldorf 1 (DE)**

EP 0 111 895 B1

## Beschreibung

Die vorliegende Erfindung betrifft eine neue Hautpflegeemulsion, welche sich insbesondere zur Anwendung auf der noch nassen Haut nach dem Baden oder Duschen, z.B. als sogenannte Duschlotion eignet.

Die menschliche Haut neigt dazu auszutrocknen und sich daher stumpf und spröde anzufühlen. Dies tritt insbesondere bei Menschen mit trockener und empfindlicher Haut auf und wird durch Waschen und Baden unter Anwendung von Seife oder synthetischen Waschaktivstoffen durch die damit verbundene Entfettung der Haut und Quellung der oberen Hautschichten noch gefördert. Es ist bekannt, diesem Übelstand dadurch abzuhelfen, daß man die Haut, insbesondere nach dem Baden oder Duschen, mit einer Hautcreme oder einer flüssigen Hautemulsion einreibt. Die Anwendung der bekannten Hautcremes und Hautpflegeemulsionen erfolgt nach dem Abtrocknen und erfordert ein sorgfältiges Einreiben oder Einmassieren der Creme oder Lotion, damit keine fettigen Rückstände auf der Haut verbleiben.

Aus DE-OS 2 243 281 sind auch bereits Hautpflegemittel bekannt, die nach dem Waschen oder Baden auf die nasse Haut aufgetragen und unter Emulgierung des Wassers in die Haut eingerieben werden können. Auch diese Art der Anwendung erfordert ein gründliches Einmassieren des Hautpflegemittels, da sich das nicht einmassierte Mittel nicht leicht von der Haut abspülen läßt.

Es bestand daher ein Bedürfnis nach einem Hautpflegemittel, welches nach dem Waschen, Baden oder Duschen auf die noch nasse Haut angewendet werden kann, das durch leichtes Verteilen auf der Haut einen pflegenden Film bildet und dessen überschüssige Anteile sich leicht mit Wasser von der Haut wieder abspülen lassen. Der pflegende Film auf der Haut soll dabei weder abgespült werden noch einen fettigen Eindruck hinterlassen.

Diese Aufgabe wurde erfindungsgemäß gelöst durch eine Hautpflegeemulsion zur Anwendung auf der nassen Haut, gekennzeichnet durch einen Gehalt an

A) einer flüssigen Ölkomponente, welche zu mindestens 20 Gew.-% aus einem flüssigen Paraffinöl, einem flüssigen Silikonöl oder einer Mischung davon besteht,in einer Menge von 5 - 30 Gew.-% der Emulsion,

B) einer Alkaliseife einer Fettsäure mit 12 - 18 C-Atomen, in einer Menge von 1 - 7 Gew.-% der Emulsion,

C) einem Alkylsulfat- und/oder Alkylpolyglykolethersulfat-Alkali- oder Magnesiumsalz mit 10 - 16 C-Atomen im Alkylrest und gegebenenfalls bis zu 12 Glykolethergruppen in einer Menge von 0,5 - 1,5 Gew.-% der Emulsion,

D) Wasser

sowie gegebenenfalls weiteren bekannten Hilfsmitteln für Hautemulsionen wie Polyolen, wasserlöslichen Polymeren, Wachskomponenten, Fettalkoholen, Komplexbildnern, Konservierungsmitteln, Duftstoffen und hautkosmetischen Wirkstoffen.

Die flüssige Ölkomponente (A) kann in einer Menge von 5 - 30 Gew.-% der Emulsion vorliegen. Sie besteht bevorzugt aus flüssigem Paraffinöl und/oder flüssigem Silikonöl, kann aber auch andere kosmetische Öle wie flüssige Fettsäuretriglyceride, flüssige Fettsäureester wie Isopropylmyristat, Isopropylpalmitat, Isopropylstearat, Oleyloleat, Decyloleat, flüssige Dicarbonsäureester wie Di-n-butyladipat, flüssige Fettalkohole wie Oleylalkohol, 2-Octyldodecanol, 2-Hexyl-decanol oder Silikonöle enthalten. Es sollte aber wenigstens 20 Gew.-% der Ölkomponenten aus flüssigem Paraffinöl und/oder flüssigem Silikonöl bestehen. Als flüssige Silikonöle sind besonders Polydimethylsiloxane und/oder Polydimethylcyclosiloxane mit einer Viskosität von 0.02-10cm$^2$/s (2-1000 Centistokes) bei + 20°C geeignet. Bevorzugt wird flüssiges Paraffinöl allein in einer Menge von 5-25 Gew.% der Emulsion als Ölkomponente verwendet.

Die Alkaliseife einer Fettsäure mit 12-18 C-Atomen (B) sollte in einer als Emulgator für die Ölkomponente (A) ausreichenden Menge vorhanden sein. Hierfür sind Mengen von 1 - 7 Gew.-% der Emulsion geeignet. Besonders günstig ist die Verwendung der Natriumseife der Palmitin- und/oder Stearinsäure in einer Menge von 2 - 4 Gew.-% der Emulsion.

Als Komponente C der erfindungsgemäßen Hautpflegeemulsion wird das Alkali- oder Magnesiumsalz eines Alkylsulfats und/oder Alkylpolyglykolethersulfats mit 10-16 C-Atomen im Alkylrest und im Falle des Ethersulfats mit bis zu 12 Glykolethergruppen in einer Menge von 0,5 bis 1,5 Gew.-% der Emulsion eingesetzt. Bevorzugt ist das Natriumsalz eines Fettalkoholethersulfats auf Basis eines Anlagerungsproduktes von 2 - 4 Mol Ethylenoxid an einen $C_{12}$-$C_{14}$-Fettalkohol in einer Menge von 0,7 bis 1,0 Gew.-% in der Emulsion enthalten.

Die erfindungsgemäßen Hautpflegeemulsionen können gegebenenfalls noch weitere für Hautemulsionen übliche Hilfsmittel enthalten, z.B. Glykole oder Polyole, wasserlösliche Polymere, Wachskomponenten, Fettalkohole, Komplexbildner, Konservierungsmittel, Duftstoffe und hautkosmetische Wirkstoffe wie z.B. UV-Filtersubstanzen, Sebostatika, Hautfeuchtigkeitsregulatoren, Pflanzenextrakte, Vitamine. Zur Verbesserung der Lagerstabilität der Emulsion und zur Erhöhung des Wasserrückhaltevermögens auf der Haut eignet sich besonders ein Zusatz eines Glykols oder Polyols aus der Gruppe Propylenglykol, Polyethylenglykol, Hexylenglykol, Glycerin, Sorbit oder Mischungen davon in einer Menge von 2 - 10 Gew.-% der Emulsion. Bevorzugt ist Propylenglykol-1.2 in einer Menge von 2 - 4 Gew.-% in der Emulsion enthalten.

Zur Erhöhung der für die Verteilung auf der Haut erforderlichen Viskosität eignet sich ein Zusatz eines wasserlöslichen Polymeren aus der Gruppe der wasserlöslichen Polysaccharide, der wasserlöslichen Polysaccharidether, der Acrylsäurepolymerisate und Copolymerisate, Polyvinylalkohol und Polyvinylpyrrolidon und/oder deren Mischungen in einer Menge von 0,1-2 Gew.-% der Emulsion. Bevorzugt werden Carboxyvinylpolymerisate - z.B. die Handelsprodukte Carbopol® - in einer Menge von 0,3 - 1,0 Gew.-% der Emulsion verwendet. Der pH-Wert der Hautpflegeemulsion sollte in einem Bereich von 7,0 bis 9,0 liegen. Bevorzugt liegt der pH-Wert der Emulsion zwischen 7,5 und 8,5.

Die erfindungsgemäßen Hautpflegeemulsionen lassen sich leicht auf der nassen Haut verteilen. Dabei bildet sich ein dünner Pflegefilm auf der Haut. Die überschüssigen auf der Haut verbleibenden Anteile der Pflegeemulsion lassen sich mühelos mit wenig Wasser von der Haut abspülen. Die bevorzugte Anwendungsform für die erfindungsgemäßen Hautpflegeemulsionen ist daher die Duschlotion, da bei dieser Anwendung mit sehr geringem Aufwand der ganze Körper einer Behandlung mit der Hautpflegeemulsion unterzogen werden kann. Selbstverständlich kann die erfindungsgemäße Hautpflegeemulsion auch als Handlotion oder Gesichtslotion auf der nassen Haut angewendet werden. Nach der Behandlung mit der Hautpflegeemulsion und dem Abspülen mit Wasser verbleibt auf der Haut ein hauchdünner Pflegefilm, der weder beim Abtrocknen noch später einen fettigen oder klebrigen Effekt verursacht. Die erfindungsgemäße Hautpflegeemulsion stellt daher eine wertvolle Bereicherung der kosmetischen Hautpflegemöglichkeiten dar.

Die Herstellung der erfindungsgemäßen Hautpflegeemulsionen erfolgt zweckmäßigerweise wie folgt:

Die Ölkomponenten A werden mit der Fettsäure, aus welcher später durch Verseifung die Komponente B entsteht und gegebenenfalls mit Wachskomponenten (z.B. Bienenwachs), Fettalkoholen (z.B. Cetyl-Stearylalkohol) gemischt und bis zum Erreichen einer klaren Schmelze erhitzt.

Die übrigen Komponenten sowie die für die Verseifung der Fettsäure vorgesehene Menge Alkalihydroxid werden im Wasser gelöst und auf ca. 8D - 90°C erwärmt. Anschließend werden die Fettphase und die wässrige Phase unter intensivem Rühren miteinander vermischt.

Die folgenden Beispiele sollen den Erfindungsgegenstand näher erläutern ohne diesen darauf zu beschränken.

## Beispiele

| Duschlotionen | Nr. 1 Gew. % | 2 Gew. % | 3 Gew. % | 4 Gew. % |
|---|---|---|---|---|
| Paraffinum perliquidum DAB | 25 | 10 | 5 | 20 |
| Isopropylatearat | — | — | 2,0 | — |
| Silikonöl (Baysilon(R) M350) | — | — | 5,0 | — |
| Bienenwachs, gebleicht | | 1,0 | | 1,0 |
| Cetyl/Stearylalkohol (50:50) | — | — | 1,0 | — |
| Palmitin-/Stearinsäure (50:50) | 3,0 | 2,0 | 3,0 | 2,0 |
| Natriumhydroxid | — | 0,5 | 0,4 | 0,5 |
| Kaliumhydroxid | 0,9 | — | — | — |
| Fallalkohol $C_{13}$ + $C_{14}$ + 3,7 EO-sulfat, NA-Salz (28 %-ig) (Texapon (R) K14S spezial) | 2,0 | 3,0 | 3,5 | 3,0 |
| Methylhydroxypropylcellulose (Culminal (R) MHPC 3000) | 1,0 | — | — | — |
| Carboxyvinylpolymerisat (Carbopol (R) 940) | — | 0,6 | 0,5 | 0,5 |
| Xantham-Gum (Keltrol (R)) | 0,2 | — | 0,2 | — |
| Ethylendiamintetraessigsäure, Na-Salz | | — | 0,2 | 0,4 |
| p-Hydroxybenzoesäuremethylester | 0,2 | 0,2 | 0,2 | 0,2 |
| p-Hydroxybenzoesäurepropylester | 0,1 | 0,1 | 0,1 | 0,2 |
| Propylenglykos V-1,2 | 2,5 | 3,0 | 5,0 | 2,8 |
| Wasser, Duftstoffe | ad 100 | ad 100 | ad 100 | as 100 |

**Patentansprüche**

1. Hautpflegeemulsion zur Anwendung auf der nassen Haut, gekennzeichnet durch einen Gehalt an

(A) einer flüssigen Ölkomponente, welche zu mindestens 20 Gew.-% aus einem flüssigen Paraffinöl, einem flüssigen Silikonöl oder einer Mischung davon besteht, in einer Menge von 5 - 30 Gew.-% der Emulsion,

(B) einer Alkaliseife einer Fettsäure mit 12 - 18 C-Atomen in einer Menge von 1 - 7 Gew.-% der Emulsion,

(C) einem Alkylsulfat- und/oder Alkylpolyglykolethersulfat-Alkali- oder Magnesiumsalz mit 10 - 16 C-Atomen im Alkylrest und gegebenenfalls bis zu 12 Glykolethergruppen in einer Menge von 0,5 bis 1,5 Gew.-% der Emulsion,

(D) Wasser sowie gegebenenfalls weiteren für Hautemulsionen bekannten Hilfsmitteln wie Polyolen, wasserlöslichen Polymeren, Wachskomponenten, Fettalkoholen, Komplexbildnern, Konservierungsmitteln, Duftstoffen und hautkosmetischen Wirkstoffen.

2. Hautpflegeemulsion gemäß Anspruch 1, dadurch gekennzeichnet, daß der pH-Wert etwa 7,0 bis 9,0, bevorzugt 7,5 bis 8,5 beträgt.

3. Hautpflegeemulsion gemäß Anspruch 1 und 2, dadurch gekennzeichnet, daß als flüssige Ölkomponente A nur ein flüssiges Paraffinöl enthalten ist.

4. Hautpflegeemulsion gemäß Anspruch 1 - 3, dadurch gekennzeichnet, daß als Alkaliseife die Natriumseife der Palmitin- und/oder Stearinsäure enthalten ist.

5. Hautpflegeemulsion gemäß Anspruch 1 - 4, dadurch gekennzeichnet, daß als Komponente C das Natriumsalz eines Fettalkoholethersulfats auf Basis eines Anlagerungsproduktes von 2 - 4 Mol Ethylenoxid an einen $C_{12}$-$C_{14}$-Fettalkohol enthalten ist.

6. Hautpflegeemulsion gemäß einem der Ansprüche 1 - 5, dadurch gekennzeichnet, daß zusätzlich die Komponenten

E) 2 - 10 Gew.-% eines Glykols oder Polyols aus der Gruppe Propylenglykol, Polyethylenglykol, Hexylenglykol, Glycerin, Sorbit und

F) 0,1-2,0 Gew.-% eines wasserlöslichen Polymeren aus der Gruppe der wasserlöslichen Polysaccharide, der wasserlöslichen Polysaccharidether, der Acrylsäurepolymerisate und Copolymerisate, Polyvinylalkohol, Polyvinylpyrrolidon enthalten sind.

7. Hautpflegeemulsion gemäß Anspruch 5, gekennzeichnet durch einen Gehalt an

5 - 25 Gew.-% eines flüssigen Paraffinöls

2 - 4 Gew.-% Na-Stearat/Palmitat

0,7 - 1,0 Gew.-% Fettalkohol $C_{12}$-$C_{14}$ + 2-4 EO-sulfat, Natriumsalz

2,0 - 4,0 Gew.-% Propylenglykol-1.2

0,3 - 1,0 Gew.-% eines Polymeren aus der Gruppe der Carboxyvinylpolymerisate

65 - 90 Gew.-% Wasser

**Claims**

1. A skin-care emulsion for application to wet skin, characterized by a content of

(A) a liquid oil component of which at least 20% by weight consists of a liquid paraffin oil, a liquid silicone oil or a mixture thereof in a quantity of from 5 to 30% by weight of the emulsion,

(B) an alkali soap of a $C_{12}$-$C_{18}$ fatty acid in a quantity of from 1 to 7% by weight of the emulsion,

(C) an alkyl sulfate and/or alkyl polyglycolether sulfate-alkali or magnesium salt containing from 10 to 16 C-atoms in the alkyl group and optionally up to 12 glycolether groups in a quantity of from 0.5 to 1.5% by weight of the emulsion,

(D) water and optionally other known auxiliaries for skin emulsions, such as polyols, water-soluble polymers, wax components, fatty alcohols, complexing agents, preservatives, perfumes and active skin-cosmetic agents.

2. A skin-care emulsion as claimed in Claim 1, characterized in that the pH-value is from about 7.0 to 9.0 and preferably from 7.5 to 8.5.

3. A skin-care emulsion as claimed in Claims 1 and 2, characterized in that only a liquid paraffin oil is present as the liquid oil component A.

4. A skin-care emulsion as claimed in Claims 1 to 3, characterized in that the sodium soap of palmitic and/or stearic acid is present as the alkali soap.

5. A skin-care emulsion as claimed in Claims 1 to 4, characterized in that the sodium salt of a fatty alcohol ether sulfate based on an adduct of from 2-4 moles ethylene oxide with a $C_{12}$-$C_{14}$ fatty alcohol is present as component C.

6. A skin-care emulsion as claimed in any of Claims 1 to 5, characterized in that the following components are additionally present:

E) from 2 to 10% by weight of a glycol or polyol from the group comprising propylene glycol, polyethylene glycol, hexylene glycol, glycerol, sorbitol and

F) from 0.1 to 2.0% by weight of a water-soluble polymer from the group comprising water-soluble polysaccharides, water-soluble polysaccharide ethers, acrylic acid polymers and copolymers, polyvinyl alcohol, polyvinyl pyrrolidone.

7. A skin-care emulsion as claimed in Claim 5, characterized in that it contains

from 5 to 25% by weight of a liquid paraffin oil,

from 2 to 4% by weight of Na-stearate/palmitate,

from 0.7 to 1.0% by weight of a $C_{12}$-$C_{14}$ fatty alcohol + 2-4 EO sulfate, sodium salt,

from 2.0 to 4.0% by weight of 1,2-propylene glycol,

from 0.3 to 1.0% by weight of a polymer from the group comprising carboxyvinyl polymers,

from 65 to 90% by weight of water.

## Revendications

1°) Emulsion pour les soins de la peau, destinée à être utilisée sur la peau humide, caractérisée en ce qu'elle contient:

(A) des huiles composantes liquides qui sont constituées d'au moins 20 % en poids d'une huile de paraffine liquide, d'une huile de silicone liquide ou d'un mélange des précédentes, dans la proportion de 5 a 30 % en poids de l'émulsion,

(B) un savon alcalin d'un acide gras à 12 à 18 atomes C, dans la proportion de 1 à 7 % en poids de l'émulsion,

(C) d'un sulfate d'alcoyle et/ou d'un polyglycoléthersulfate d'alcoyle, sel de métal alcalin ou de magnésium, avec 10 à 16 atomes de carbone dans le reste alcoyl, et éventuellement jusqu'à 12 groupes glycoléther, dans la proportion de 0,5 à 1,5 % en poids de l'émulsion,

(D) de l'eau, ainsi qu'éventuellement d'autres produits auxiliaires connus pour les émulsions pour la peau, tels que des polyols, polyméres solubles à l'eau, composants cireux, alcools gras, complexants, agents de conservation, parfums et substances ayant une action cosmétique sur la peau.

2°) Emulsion suivant la revendication 1, caractérisée en ce que le pH est d'environ 7 à 9, de préférence de 7,5 à 8,5.

3°) Emulsion suivant les revendications 1 et 2, caractérisée en ce qu'il y est contenu comme composant huile A, seulement de l'huile de paraffine liquide.

4°) Emulsion suivant les revendications 1 à 3, caractérisée en ce qu'elle contient, comme savon alcalin, le savon de sodium de l'adide palmitique et/ou stéarique.

5°) Emulsion suivant les revendications 1 à 4, caractérisée en ce qu'il est contenu, comme composant C, le sel de sodium d'un éthersulfate d'alcool gras, à base d'un produit d'addition de 2 à 4 moles d'oxyde d'éthylène sur un alcool gras en $C_{12}$ à $C_{14}$.

6°) Emulsion suivant l'une des revendications 1 à 5, caractérisé en ce qu'elle contient, supplémentairement, les composants:

E) 2 à 10 % en poids d'un glycol ou polyol du groupe propylèneglycol polyéthylèneglycol, hexylèneglycol glycérine, sorbite, et

F) 0,1 à 2 % en poids d'un polymère soluble à l'eau, du groupe des polysaccharides solubles à l'eau, des éthers polysaccharidiques solubles à l'eau, des polymérisats et copolymérisats de l'acide acrylique, d'alcool polyvinylique, de polyvinylpirrolidone.

7°) Emulsion suivant la revendication 5, caractérisée en ce qu'elle contient:

5 à 25 % en poids d'une huile de paraffine liquide

2 à 4 % en poids d'un stéarate/palmitate de Na

0,7 à 1 % en poids d'alcool gras en $C_{12}$ à $C_{14}$ + 2 à 4 sulfate d'EO, sel de sodium,

2 à 4 % en poids de propylèneglycol-1,2,

0,3 à 1 % en poids d'un polymère du groupe des polymérisats de carboxyvinyle 65 à 90 % en poids d'eau.